Europäisches Patentamt

(19) European Patent Office

Office européen des brevets

(11) Publication number: **0 239 776 B1**

## (12) EUROPEAN PATENT SPECIFICATION

(45) Date of publication of patent specification: **19.08.92**   (51) Int. Cl.5: **G01N 33/569**, G01N 33/577

(21) Application number: **87102484.0**

(22) Date of filing: **21.02.87**

(54) **Reagent containing monoclonal antibodies to Bacteroides gingivalis.**

(30) Priority: **26.02.86 US 833849**

(43) Date of publication of application:
**07.10.87 Bulletin 87/41**

(45) Publication of the grant of the patent:
**19.08.92 Bulletin 92/34**

(84) Designated Contracting States:
**AT BE CH DE ES FR GB GR IT LI LU NL SE**

(56) References cited:

**CHEMICAL ABSTRACTS, vol. 103, no. 19, 11
November 1985, Columbus, OH (US);
Y.NAITO et al., p. 554, no. 158787s**

**CHEMICAL ABSTRACTS, vol. 103, no. 7, 19
August 1985, Columbus, OH (US); LION
CORP., p. 292, no. 50873c**

**BIOLOGICAL ABSTRACTS, vol. 83, 1987,
Philadelphia, PA (US); P.CHEN**

**CHEMICAL ABSTRACTS, vol. 105, no. 11, 15
September 1986, Columbus, OH (US);
T.MIYOSHI et al., p. 490, no. 95766c**

(73) Proprietor: **THE RESEARCH FOUNDATION OF
STATE UNIVERSITY OF NEW YORK
State University Plaza, Broadway
Albany, New York 12246(US)**

(72) Inventor: **Genco, Robert Joseph
32 St. Catherine's Court
Buffalo, NY 14222(US)**
Inventor: **Christersson, Lars Anders
203 Washington Highway
Snyder, NY 14226(US)**
Inventor: **Chen, Priscilla B.
5640 Davison Road
Clarence, NY 14031(US)**
Inventor: **Zambon, Joseph James
133 President's Walk
Williamsville, NY 14221(US)**
Inventor: **Neiders, Mirdza Erika
181 Smallwood Drive
Amherst, NY 14226(US)**

(74) Representative: **Weber, Dieter, Dr. et al
Dr. Dieter Weber und Dipl.-Phys. Klaus Seif-
fert Patentanwälte Gustav-Freytag-Strasse
25 Postfach 6145
W-6200 Wiesbaden 1(DE)**

Rank Xerox (UK) Business Services

BIOLOGICAL ABSTRACTS, vol. 81, 1986, Philadelphia, PA (US); Y.NAITO

BIOLOGICAL ABSTRACTS, vol. 81, 1986, Philadelphia, PA (US); L.G.SIMONSON et al.

BIOLOGICAL ABSTRACTS/RRM, vol. 31, 1986, Philadelphia, PA (US); P.CHEN et al.

INFECT. IMMUN, vol. 54, no. 3; P.CHEN et al., pp. 798-803

**Description**

The present invention relates to monoclonal antibodies and antigens, and to the use of such antibodies and antigens in the detection of the presence and concentration of certain specific microorganisms implicated in the etiology of human periodontal disease. More specifically, the present invention relates to monoclonal antibodies specific to antigens from Bacteroides gingivalis and is generally associated with adult periodontal disease.

Clinical assays specific to such bacteria in gingival and subgingival dental plaque are useful in the diagnosis of periodontal disease, in evaluating the progress of periodontal therapy, and in determining the status of the patient at subsequent recall examinations. The standard bacteriological techniques presently in use to identify such microorganisms are time consuming, expensive, and require a high level of expertise. Further, such tests frequently give results which are not as accurate or as sensitive as desired or required.

Recently, various efforts and proposals have been made to improve or replace the standard bacteriological techniques by the utilization of immunodiagnostic assay techniques. Such techniques are based upon the formation of a complex between the antigenic substance being assayed and an antibody or antibodies in which one or the other member of the complex is labeled. For example, labeling may be by means of a radioactive element, such as $I^{125}$, (radioimmunoprecipitation assays); a material having fluorescent properties (immunofluorescence assays); enzyme-linked immunoabsorbant assays (ELISA) or (immunoperoxidase assays). The labeled member of the complex facilitates detection and/or qualitative analysis of the complexed labeled antigen or antibody from the uncomplexed antigen or antibody.

In typical competition utilizing immunoassay assay techniques, the antigenic substance in the sample of the fluid being tested competes with a known quantity of labeled antigen for a limited quantity of antibody binding sites. Thus, the amount of labeled antigen bound to the antibody is inversely proportional to the amount of antigen in the sample. In contrast, typical immunometric assays employ a labeled antibody. In such assay, the amount of labeled antibody associated with the complex is directly proportional to the amount of antigenic substance in the fluid sample.

Although immunodiagnostic techniques can be, a substantial improvement over presently used bacteriological detection techniques, many further improvements remain to be made. For example, the immunodiagnostic techniques presently in use frequently produce variable results because relatively crude antigens and antibodies are employed. For example, three serotypes of Actinobacillus actinomycetemcomitans are known to exist in the oral cavity of man, yet one or more of the serotypes frequently goes undetected using present immunodiagnostic techniques. Similarly, there are at least three serologic variants of B. gingivalis in the oral cavity of man and they differ in virulence, hence the identification of the serologic variants of B. gingivalis (group A represented by strain 381 and group B represented by strains W50 and SAC7A1-28) by immunologic means can offer considerable advantages over other methods of detection and quantitations, however, present methods do not allow such distinction to be made. The immunodiagnostic techniques which are currently available for use in the determination of periodontal diseases generally lack accuracy, sensitivity and specificity.

Monoclonal antibodies may be defined as identical proteins produced by known techniques. The basic techniques for producing monoclonal antibodies were developed in the mid-1970's after Kohler and Milstein successfully fused spleen lymphocytes with malignant cells (myelomas) of bone marrow primary tumors [C. Milstein, Sci Am. 243(4): 66-74, (1980)]. Monoclonal antibodies recognize a single specific antigenic determinant (chemical structure). A given antigen may have several determinants. Thus, if monoclonal antibodies alone are utilized, immunodiagnostic tests may lack specificity because the same determinant may be found on different antigens or molecules, or false reading may be made because a single antigen may contain repeating determinants. Conventionally produced antigens typically have multiple determinants. Until the present invention, efforts to solve this problem have been made without a great deal of success. It is highly desirable that monoclonal antibodies be produced that not only have the ability to recognize a desired molecule or structure in a complex, but also have the ability not to recognize the same determinant in an unrelated or undesired complex.

Chemical Abstracts, volume 103, number 7 (1985), abstract number 50 837c, discloses that a diagnosis of periodontal disease is possible by means of antibodies to Bacteroides gingivalis. Chemical Abstracts, volume 103, number 19 (1985), abstract number 158 787s, shows the existence of monoclonal antibodies against surface antigens of Bacteroides gingivalis, and states that the tested give monoclonal antibodies reacted with sonicated antigen from all Bacteroides gingivalis strains tested so that these antibodies were apparently not capable of detecting feature differentiating between different types of those bacteria.

The immunodiagnostic technics which are currently available for use in the determination of the periodontal disease generally lack accuracy, sensitivity and specificity.

3

Thus, the objective was to find an immunodiagnostic method which allows to differentiate between different types of Bacteroides gingivalis bacteria, especially those of different virulence.

This objective is solved according to the invention by a monoclonal antibody capable of detecting a feature differentiating between different types of Bacteroides gingivalis bacteria and having the deposition number of In Vitro International, Inc. IVI-10102 (BBG-12), IVI-10103 (BBG-30), IVI-10104 (BBG-31) or IVI-10105 (BBG-42).

The present invention provides a specific antigen for microorganisms which are associated with periodontal diseases and monoclonal antibodies that recognize only those antigens, and more particularly monoclonal antibodies that recognize such antigens. Thus the present invention provides a means of substantially improving the accuracy of various immuno-diagnostic assays.

Specific microorganisms have been strongly associated in the etiology of human periodontal disease and Bacteroides gingivalis frequently has been implicated in adult periodontitis. The present monoclonal antibodies are specific to Bacteroides gingivalis.

The present monoclonal antibodies are typically employed as a reagent which includes an inert carrier, preferably a liquid, such as buffered saline solution and a preservative. The carrier compositions are selected to provide for the proper dispersal of bacteria, and to preserve the integrity of antigens and supplemental structures. The selection of the proper carrier is also important in the detection of bacteria which produce large amounts of autolytic enzymes such as B. gingivalis.

The monoclonal antibodies of the present invention are useful in clinical testing and differentiating antigens in the gingival or subgingival sera. The method of testing and differentiating involves the steps of contacting a sample of the bacterial flora from a lesion or other site with a measured amount of a monoclonal antibody specific to a single antigen site on Bacteroides gingivalis and subsequently measuring the number of antibody-antigen complexes formed.

The monoclonal antibodies useful in the present invention are obtained by the process discussed by Milstein & Kohler and reported in Nature, 256, 495-97, 1975. The details of the process are well known in the art. Basically, the process involves injecting a mouse, or other suitable animal, with an immunogen. The animal is subsequently sacrificed and cells taken from its spleen and fused with myeloma cells. The result is a hybrid cell, hybridoma, that reproduces in vitro. The population of hybridomas are screened to isolate individual classes each of which secrete a single antibody specific to the antigen. The individual antibody species obtained in this way are each the product of a single B cell from the immune animal generated in response to a specific antigenic site recognized by the immunogenic substance. The monoclonal bodies selected preferably have a response affinity of at least $10^7$ liters/mole and, more preferably, an affinity of at least $10^9$ liters/mole.

The invention is also directed to a method of determining the presence of special types of Bacteroides gingivalis in a gingival fluid comprising the steps of

a) contacting the gingival fluid with a monoclonal antibody having the deposition number of In Vitro International, Inc. IVI-10102 (BBG-12), IVI-10103 (BBG-30), IVI-10104 (BBG-31) or IVI-10105 (BBG-42) to form complexes of said antigenic substance and said monoclonal antibody, and

b) measuring the amount of said complex by determining the amount of antibody in said formed complex.

Further the invention refers to a method of determining the presence of special types of Bacteroides gingivalis in a biological fluid comprising the steps of

a) contacting an aliquot of the biological fluid to be studied with a measured amount of antibodies having the deposition number of In Vitro International, Inc. IVI-10102 (BBG-12), IVI-10103 (BBG-30), IVI-10104 (BBG-31) or IVI-10105 (BBG-42), and

b) determining whether any reaction with said antibodies occurs.

In this latter method, preferably, the antibodies are labeled and said labeled antibodies have a predetermined fluorescence response to a given optical stimulation.

Preferably, the amount of reaction is determined and the presence of periodontopathogens is quantitatively derived therefrom, especially said determination is made by ELISA after lysing the suspension by a solubilizing solution.

Bacteroides gingivalis is a highly anaerobic microorganism the culture of which requires strict anaerobiosis from the time of sample taking through sample dispersion, plating and culturing. For these reasons B. gingivalis is difficult to reliably quantitate in clinical samples. Bacteroides gingivalis is commonly isolated from subgingival pockets with progressing alveolar bone loss. Its primary oral ecological niche appears to be periodontal pockets.

Using rabbit antisera to a series of strains of Bacteroides gingivalis of human oral origin, B. gingivalis was found to possess a set of antigens which are antigenically unique compared to the other black-

4

pigmented Bacteroides including Bacteroides asaccharolyticus, Bacteroides melaninogenicus, Bacteroides levii, Bacteroides loeschii, and Bacteroides intermedius (C. Mouton, et al. Ann Microbiol. (Inst. Pasteur), 132B: 69-83, 1981). Such antigens were also found to be absent from a battery of other gram-negative and gram-positive oral microorganisms. There at lease two antigenic types of B. gingivalis which differ in virulence and to which rabbit antisera and monoclonal antibodies have been prepared. Serotype a is represented by strain 381 and serotype b by strains W50, W83, and SAC7A1-28. The latter strain shows an extremely high level of virulence in experimental abscess formation. The present monoclonal antibodies allow the immunologic detection of Bacteroides gingivalis and the identification of antigenically different strains which differ in virulence by the use of specific antibodies.

Monoclonal antibodies then were produced to Bacteroides gingivalis and screened for reactivity with various antigenic preparations of B. gingivalis by the following procedures. BALB/c mice were immunized with whole cells of Bacteroides gingivalis strain 381. The spleens were removed and cells fused with SP-2 myeloma cells using polyethylene glycol (PEG-1000). The desired hybrid cells were selected by adding hypozanthineaminopterin-thymidine to the medium. Surviving hybrid clones in individual wells of microtiter plates were tested for antibody production using the ELISA assay with whole cells of B. gingivalis as the antigen. All of the clones were assayed against whole cells of Bacteroides gingivalis, and 30 were found to react specifically with Bacteroides gingivalis whole cells and to be unreactive when tested against a large battery of oral gram-positive or gram-negative organisms.

A set of clones produced antibodies which differentiate among serotypes of B. gingivalis which vary in virulence. The clones are directed to serologic antigens (SA) and are Clones BBG-12 and BBG-42. Their reactivity with "virulent" (W50) and "less virulent" (381p) strains of B. gingivalis is shown in Table 1.

Table 1

| Clones Which Differentiate Virulent Strains of B. Gingivalis | | |
|---|---|---|
| | B. Gingivalis 381p | B. Gingivalis W50 |
| Clone BBG-12 | 3(a) | 4 |
| Clone BBG-42 | 4 | - |

(a) Scored by indirect immunofluorescence on whole cells of representative strains of B. gingivalis.

Other clones were found by Western blot techniques to react with a 43K fimbriae component of B. gingivalis. These clones, BBG-30 and BBG-31, exhibit high affinity to B. gingivalis fimbriae, which is the 43K antigen, and were found to react strongly with B. gingivalis cells in indirect immunofluorescent tests. These antibodies may also be useful in the differentiation of the virulent strains since they appear unique to each of the 43K fimbriae antigens.

The use of these monoclonal antibodies to 43K fimbriae, and Clones BBG-12 and BBG-42 which differentiate serotype (SA) antigens, in indirect immunofluorescent tests on slides with bacterial smears taken from periodontal lesions shows that they all react with cell surface components of a microorganism with the cellular morphology of Bacteroides gingivalis and with no other cellular morphotype of the many morphotypes present in the bacterial smear from periodontal lesions. The reaction was identical to that seen on smears of pure cultures of B. gingivalis alone, or on smears of pure cultures of B. gingivalis artificially mixed with pure cultures of a number of other oral microorganisms. Hence, the testing of bacterial samples taken from periodontal lesions is consistent with the specificity determined in ELISA, immunoblot, Western blot, immunofluorescence and gel precipitation tests.

Immunofluorescence assays using Clones BBG-12, BBG-30, BBG-31, and BBG-42 used singly or in combination detect unique surface antigens of B. gingivalis found in suspensions of the microorganism in the presence of a large number of other microorganisms and are useful in the detection and quantitation of B. gingivalis samples taken from lesions.

Clones BBG-12, BBG-30, BBG-31, and BBG-42 are deposited and registered with In Vitro International, Inc., 7885 Jackson Road, Ann Arbor, MI.

Testing

The monoclonal antibodies of the present invention were tested in a series of immunologic assays and were shown to be specific for the detection of microorganisms to which they are directed. In such tests

5

Bacteroides gingivalis, antibody Clones BBGH-12, BBG-30, BBG-31, and BBG-42 were used in immunoblot, Western bloc, ELISA, F-ELISA and immunofluorescence analysis. The results demonstrate that the above listed clones are specific to Bacteroides gingivalis with no observable reactivity to other microorganisms from the test panel. Furthermore, in the immunofluorescent assay these antibodies react specifically with a cellular morphotype consistent with Bacteroides gingivalis, when tested in the presence of large numbers of other bacterial cellular morphotypes. Such specificity seen with the present monoclonal antibodies is in sharp contrast to the often strong and unpredictable cross-reactions seen with the given art polyclonal sera obtained from mice, rabbits, goats and guinea pigs. Further, although the commonly employed methods may obtain highly specific polyclonal antisera, when that particular bleeding or batch is depleted, another batch must be prepared, absorbed and tested, and even then the results with the new batch may not match that obtained with the first batch. Much effort is needed to prepare a batch of polyclonal antibodies with the necessary high titer and specificity. The present monoclonal antibodies offers the substantial advantage of consistency and availability. The present hybridomas have been repeatedly subcultured and have shown to give rise to copious ascites fluid in mice.

Immunofluorescence Microscopy

Clinical studies were performed comparing the use of monoclonal antibodies to Bacteroides gingivalis with bacterial culture of these microorganisms. Twenty subjects were examined for Bacteroides gingivalis including 10 normal adults and 10 adult periodontitis patients. As seen in Table 2, immunofluorescence microscopy using monoclonal antibody Clone BBG-12 was more likely to detect Bacteroides gingivalis that bacterial culture on selective or nonselective media, especially when this microorganism was present in small numbers in subgingival dental plaque. The monoclonal antibody detected the microorganism in subgingival plaque samples in adult periodontitis patents that could not be seen by culture on nonselective media.

There was found to be a correlation between indirect inmunofluorescence microscopy for the detection of B. gingivalis using monoclonal antibody (for example, Clone BBG-12), which is representative of the other species specific clones, and culture on selective and nonselective media. For this organism it was found that to obtain accurate results the sample must either be processed within a few hours, or preserved in a transport media. A viable transport media (TM) has been found to consist of from about 0.1 to about 0.2M of NaCl, from about 0.01 to about 0.03M of $NaPO_4$, and from about 0.5 to about 2.0 percent by weight formalin and buffered to a pH of about 7.4. A particularly preferred TM consists of 0.15 M NaCl, 0.02 M $NaPO_4$, 2% formalin at pH 7.4. Otherwise, antigen deterioration and agglutination occurred. A number of other media containing various buffer systems and preservatives such as paraformaldehyde and gluteraldehyde were tried and found not to be useful as TM. Bacterial samples from lesions are stable in TM for at least six months at room temperature according to our methods studies. Table 3 shows that immunofluorescence microscopy can detect B. gingivalis better than culture when this organism is present in small numbers in subgingival plaque. In adult periodontitis patients, approximately twice the number of subgingival plaque samples were shown to harbor B. gingivalis by immunofluorescence using monoclonal antibody as compared to culture on either selective or nonselective media.

Table 2

| Presence of B. Gingivalis in Subgingival Plaque | | | | | |
|---|---|---|---|---|---|
| Subject Group | Subjects Examined | Sites Examined | Culture | | Immunofluorescence |
| | | | Non-selective Media | Selective Media | Monoclonal Antibody (BBG-12) |
| Normal Subjects | 10 | 60 | 0(0) | 0(0) | 1(2) |
| Adult Periodontitis Patients | 10 | 60 | 12(20) | 13(21) | 28(43) |
| TOTAL | 20 | 120 | 12(10) | 13(11) | 19(25) |

The sensitivity of monoclonal antibody for detection of B. gingivalis compared to culture on nonselective media is 92%, the specificity is 88%, the predictive value of a positive test is 38%, and the predictive value of a negative test is 99%. Comparing immunofluorescence microscopy using monoclonal antibody with

6

culture on selective media the sensitivity is 91%, specificity is 89%, the predictive value of a positive test is 35%, and the predictive value of a negative test is 96%.

Indirect immunofluorescence microscopy using monoclonal antibodies for the detection of B. gingivalis in subgingival dental plaque is useful in the diagnosis of human periodontal disease.

The sensitivity of the immunofluorescence assays is great since theoretically a single strongly stained B. gingivalis cell can be detected among hundreds of thousands of bacteria.

Flow Cytometry

The enumeration of stained microorganisms may be done manually by reading and counting. It was found that automation of immunofluorescence using a Cytofluorograph, a product of Ortho Instruments, Westwood, MA, results in an objective, rapid, enumeration of microorganisms. Other methods of automation such as use of image analysis are useful in reducing the labor necessary for enumerating the percent of stained specific organisms.

Bacterial samples (approximately $10^8$ bacteria/ml) of B. gingivalis and/or B. intermedius were washed 3x with phosphate buffered saline (PBS), pelleted, resuspended in 100 microliter of a 1:6 dilution of ascites fluid containing monoclonal antibody to (Clone BBG-12) B. gingivalis and incubated at 37°C for 30 minutes. The cells were washed 3x with PBS, pelleted, resuspended in 100 microliter of optimally diluted fluorescein-conjugated goat anti-mouse IgG, and incubated at 37°C for 30 minutes. The cells were again washed 3x with PBS, pelleted, resuspended in 1.0 ml PBS and passed through an 18 gauge needle equipped with a 52 micron mesh filter. Aliquots were placed on microscope slides for quantitation of labeling by fluorescence microscope techniques. The remaining aliquots were evaluated by flow cytometry using an Ortho 50H Cytofluorograph. It was necessary to hold these samples in the transport media described above.

Analysis of the labeled bacteria by flow cytometry demonstrated positive staining of greater than 95% of the target population (Table 3), and which correlated with immunofluorescence microscopy using the same reagents (Table 4). Nonspecific staining, as shown by incubation of the bacteria with the Miles conjugate was 64.4%, while autofluorescence of bacteria was acceptably low at 1.9%. The percentage of cells staining nonspecifically was not dramatically different from control positives, but there were variations in the peak (where there are the greatest number of cells at one particular fluorescent intensity) and mean channel values between positive and negative controls. Both of the negative controls demonstrated a peak at channel 8. The positive controls peak was between channels 14 and 41 dependent upon the dilution of monoclonal antibody. The means for the positive cultures ranged from a mean channel intensity of 59.8 to 138.9. Optimum monoclonal staining was between the 1:16 dilution where the peak channel was 41 with a mean channel intensity of 90.7 and the 1:32 dilution where the peak channels was 39 with a mean channel intensity of 137.9.

## Table 3

### Flow Cytometric Analysis of B. Gingivalis

| Bacteria | Primary Antibody Clone BBG-12 Dilution | Secondary Antibody F-a Mouse Ig Dilution | Percentage Particles Fluorescing[a] |
|---|---|---|---|
| 1) B. gingivalis | - | - | 1.9 |
| 2) " | - | 1:30 | 64.6[b] |
| 3) " | 1:8 | 1:30 | 95.7 |
| 4) " | 1:16 | 1:30 | 98.8 |
| 5) " | 1:32 | 1:30 | 98.5 |
| 6) " | 1:64 | 1:30 | 96.9 |

(a) Calculated as $\dfrac{\text{No. of particles fluorescing}}{\text{Total No. of particles in a gated area}}$

(b) The intensity of fluorescence of these particles was 1/3 of that of the samples treated with both primary and secondary antibodies, and hence can be distinguished from the true specific reactions in 3, 4, 5 and 6.

Fluorescent microscopic analysis or these samples (Table 4) demonstrated only slight variation in positive and negative controls. At dilutions of 1:16 and 1:32, there were 3 + reactions of labeled bacteria.

## Table 4

### Immunofluorescence Microscopic Analysis of B. gingivalis

| Bacteria | Primary Antibody Clone BBG-12 Dilution | Secondary Antibody F-a Mouse Ig Dilution | Bacterial Cells Fluorescing |
|---|---|---|---|
| B. gingivalis | - | - | - |
| " | - | 1:30 | - |
| " | 1:8 | 1:30 | + |
| " | 1:16 | 1:30 | +++ |
| " | 1:32 | 1:30 | +++ |
| " | 1:64 | 1:30 | + |

8

Flow cytometric analysis of mixtures of B. gingivalis and B. intermedius in liquid medium demonstrated variable percentages of positive fluorescence, paralleling the relative changes in number or target cells (B. gingivalis vs. B. asaccharolyticus) seen by immunofluorescence microscopy (Table 6). It can be seen that nonspecific reactions, e.g. B. gingivalis stained by the second antibody, or B. asaccharolyticus stained by both antibodies has a low "peak" channel (85-110) which indicates a low staining intensity. On the other hand, the five positive reactions have high peak values (376 to 987). Hence, the flow cytometric method allows one to consider only strong reactions (peaks ≥ 300) as true positives. When this is done, the mixtures of B. gingivalis and B. asaccharolyticus stain nearly as theoretically expected (last three rows in Table 5).

Table 5

Flow Cytometric and Slide Immunofluorescent Comparative Analysis of Artificial
Mixtures of B. Gingivalis and a Non-cross-reacting Bacteroides, B. Asaccharolyticus

| Bacteria | Anti-B. Gingivalis Monoclonal Antibody (1/16 Dilution) | 2nd Antibody (FITC Goat Anti-Mouse) (1/30 Dilution) | Flow Cytometry | | Indirect Immunofluorescence Microscopy | |
|---|---|---|---|---|---|---|
| | | | % Positive[1] | Peak[2] | IF[3] | % Positive[4] |
| B. Gingivalis | - | - | 0 | - | 0 | |
| B. Gingivalis | - | + | 1.3 | 110 | 0 | |
| B. Asaccharolyticus | - | - | 0 | - | 0 | |
| B. Asaccharolyticus | - | + | 4.2 | 85 | 0 | |
| B. Gingivalis | + | + | 84.6% | 366 | 3+ | |
| B. Asaccharolyticus | + | + | 29.0% | 90 | 1+ | |
| B. Gingivalis (10%)[5] + B. Asacc. (90%) | + | + | 4.5% | 987 | 4+ | 14% ± 2.8% |
| B. Gingivalis (50%)[5] + B. Asacc. (50%) | + | + | 51.0% | 725 | 3+ | 50% ± 16.5% |
| B. Gingivalis (90%)[5] + B. Asacc. (10%) | + | + | 86.7% | 376 | 3+ | 75% ± 7.0% |

1. Number of fluorescent-positive cells/total number of cells counted.
2. Peak = channel number where greatest number of fluorescent-positive cells were seen.

### Immunofluorescence Microscopy Assessment:
3. 3+ = moderate fluorescence, with good cell envelope definition.
   4+ = brilliant fluorescence, with good cell envelope definition.
4. Number of cells fluorescent-positive/total number of cells with microscope field; mean of 3 fields.
5. Mixtures of B. gingivalis and B. asaccharolyticus.

<u>SUNY Modified Antigen-Capture ELISA Protocol</u>

Rabbit anti-<u>B. gingivalis</u> 381

Solid Phase

1:1000 dilution in antigen buffer
(0.1M carbonate, bicarbonate,
0.2% NaN$_3$, pH 9.6)
150 microliter/well

4 hours room temp.
humid chamber

3% bovine serium albumin (BSA)
in antigen buffer
150 microliter/well

18 hours 4°C

wash 2x phosphate buffered saline (PBS),
1x antibody + conjugate buffer

Antigen

Bacteria in 0.05% sodium dodecyl
sulfate (SDS), undiluted 10$^{-3}$
in Antibody + conjugate buffer
(PBS, 1% BSA, 0.5% Tween 20,
.02% NaN$_3$, pH 7.2)
100 microliter/well

2 hours room temp.
shaking

wash as above

Antibody

murine monoclonal antibody to <u>B. gingivalis</u>
1:1000 dil. in antibody
+ conjugate buffer
100 microliter/well

2 hours room temp.
humid chamber
99% humidity

wash as before

Conjugate

goat anti-mouse IgG conjugated
with alkaline phosphatase
1:1000 dil. in antibody
+ conjugate buffer
100 microliter/well

18 hours room temp.
humid chamber
99% humidity

wash 2x PBS, 1 x substrate buffer

<u>Enzyme-linked Immunosorbent Assays</u>

The sensitivity of the ELISA assay may be increased to determine microorganisms in the range of 5 x 10$^4$ organisms per ml using the following modifications:

1. The bacterium are lysed using 0.05% sodium dodecyl sulfate (SDS) to release antigens in soluble form. The soluble antigens are assayed by using ELISA, F-ELISA or immunoblot. (This treatment was found to increase the sensitivity 100 fold, increasing the minimum levels for detection of organisms from one million to 1000 per ml.)

2. The ELISA and F-ELISA assays are carried out using the "antigen capture" method whereby polyclonal antibody (or another monoclonal antibody) monospecific to the bacterium is absorbed to a well, the antigen is added, and then antibody to the monoclonal antibody (or a second monoclonal antibody if monoclonal antibody is used in the first step) which is conjugated with alkaline phosphatase. The sensitivity of detection is in the range of $10^4$ organisms/ml.

3. In the use of the fluorescent ELISA assay the light adsorbing ligand 2-naphtoyltrifluoroacetone is added and the sensitivity is increased approximately another tenfold by monitoring the production of a fluorescent product in a fluorescent ELISA reader.

4. The use of multiple steps for the detection including the use of avidin or strepsvidin and biotin; "multiple layered sandwiches" using goat anti-mouse IgG subclass sera, and rabbit anti-goat sera, which is conjugated with alkaline phosphatase.

These modifications are useful to increase the sensitivity of the assays which may be necessary for use where few cells are available for sampling such as after therapy or in the saliva. After therapy for example, there are often $10^6$ organisms recoverable from a lesion. If B. gingivalis or A. actinomycetemcomitans are present at 1% of the total cell population, this requires a sensitivity of approximately $10^4$ cells per ml for accurate determination.

The labeled monoclonal antibodies of the present invention may be provided with the same labels used in the immunometric assays. Among those are fluorogenic labels for detection by fluorimetry as described in U.S. Patent 3,940,475, enzymatic markers as described in U.S. Patent 3,645,090, or radioisotopes as described by Hunter and Greenwood, Nature, 144: 945, 1962.

The present invention contemplates the use of various test procedures known in the art to determine the presence of bound antibodies in fluids. Such tests include, utilization of plastic surfaces, such as polyvinyl chloride, polystyrene, glass surfaces, and nitrocellulose on which the antigen is coated.

The present antibodies may suitably and preferably be utilized with other antibodies in a monoclonal antibody reagent useful in determining periodontopathogens. For example, Actinobacillus actinomycetemcomitans is generally associated with juvenile periodontitis and Bacteroides gingivalis is generally associated with adult periodontitis, however, a preferred method would be to test for both pathogens in each patient.

Sampling Kit

For clinical use of the monoclonal antibodies in immunologic diagnosis of periodontal organisms, a testing means in the form of a kit may be used to provide a convenient method of sampling of the subgingival flora, and an effective means of transporting the sample to a reference library where the assays would be performed. In order to insure a standardized sample taking procedure clear instructions should be included in the kit. The kit suitably includes paper points 15-20 mm long with a stiff handle. These may be supplied in a sterile packet with instructions to place in a prepared subgingival site for 10 seconds.

An important component of the kit is the transport media (TM). Our studies have shown that antigens of B. gingivalis and to some extent A. actinomycetemcomitans are destroyed by storing at room temperature in aqueous buffer. A particularly useful TM has been found to consist of from about 0.1 to about 0.2M NaCl, from about 0.01 to about 0.03M NaPO$_4$ phosphate buffered to a pH of about 7.2, containing from about 0.5 to about 3.0% by weight Formalin (v/v). This transport media preserves the antigens for periods of up to six months. This media also favors adequate dispersal of the bacterial mass from the paper points so that usable suspensions on smears in which clumping and aggregation is minimal can be made or slides for immunofluorescent analysis, or for cytofluorometric analysis. Furthermore, this media allows for solubilization of the organism for ELISA and other assays using 0.05% SDS added to the transport media described above.

**Claims**

1. A monoclonal antibody capable of detecting a feature differentiating between different types of Bacteroides gingivalis bacteria and having the deposition number of In Vitro International, Inc. IVI-10102 (BBG-12), IVI-10103 (BBG-30), IVI-10104 (BBG-31) or IVI-10105 (BBG-42).

2. The monoclonal antibody of Claim 1 wherein the feature is serotype and the monoclonal antibody is IVI-10102 (BBG-12) or IVI-10105 (BBG-42).

3. The monoclonal antibody of Claim 1 wherein the feature is the presence of fimbriae and the monoclonal antibody is IVI-10103 (BBG-30) or IVI-10104 (BBG-31).

4. A method of determining the presence of special types of Bacteroides gingivalis in a gingival fluid comprising the steps of
   a) contacting the gingival fluid with a monoclonal antibody having the deposition number of In Vitro International, Inc. IVI-10102 (BBG-12), IVI-10103 (BBG-30), IVI-10104 (BBG-31) or IVI-10105 (BBG-42) to form complexes of said antigenic substance and said monoclonal antibody, and
   b) measuring the amount of said complex by determining the amount of antibody in said formed complex.

5. A method of determining the presence of special types of Bacteroides gingivalis in a biological fluid comprising the steps of
   a) contacting an aliquot of the biological fluid to be studied with a measured amount of antibodies having the deposition number of In Vitro International, Inc. IVI-10102 (BBG-12), IVI-10103 (BBG-30), IVI-10104 (BBG-31) or IVI-10105 (BBG-42), and
   b) determining whether any reaction with said antibodies occurs.

6. The method of Claim 5 wherein the antibodies are labeled and said labeled antibodies have a predetermined fluorescence response to a given optical stimulation.

7. The method of Claim 5 wherein the amount of reaction is determined and the presence of periodontopathogens is quantitatively derived therefrom.

8. The method of Claim 5 wherein said determination is made by ELISA after lysing the suspension by a solubilizing solution.

**Patentansprüche**

1. Monoklonaler Antikörper, der in der Lage ist, ein zwischen unterschiedlichen Bakterientypen von Bacteroides gingivalis differenzierendes Merkmal festzustellen und mit der Hinterlegungsnummer von In Vitro International, Inc. IVI-10102 (BBG-12), IVI-10103 (BBG-30), VIV-10104 (BBG-31) oder IVI-10105 (BBG-42).

2. Monoklonaler Antikörper nach Anspruch 1, bei dem das Merkmal ein Serotyp ist und der monoklonale Antikörper IVI-10102 (BBG-12) oder IVI-10105 (BBG-42) ist.

3. Monoklonaler Antikörper nach Anspruch 1, bei dem das Merkmal das Vorhandensein von Fimbrien ist und der monoklonale Antikörper IVI-10103 (BBG-30) oder IVI-10104 (BBG-31) ist.

4. Verfahren zur Bestimmung des Vorhandenseins spezieller Typen von Bacteroides gingivalis in einer gingivalen Flüssigkeit mit den Stufen, in denen man
   a) die gingivale Flüssigkeit mit einem monoklonalen Antikörper, der die Hinterlegungsnummer von In Vitro International, Inc. IVI-10102 (BBG-12), IVI-10103 (BBG-30), IVI-10104 (BBG-31) oder IVI-10105 (BBG-42) hat, unter Bildung von Komplexen der Antigensubstanz und des monoklonalen Antikörpers behandelt und
   b) die Menge des Komplexes durch Bestimmung der Antikörpermenge in dem gebildeten Komplex mißt.

5. Verfahren zur Bestimmung des Vorhandenseins spezieller Typen von Bacteroides gingivalis in einer biologischen Flüssigkeit mit den Stufen, in denen man
   a) einen Anteil der zu untersuchenden biologischen Flüssigkeit mit einer abgemessenen Menge von Antikörpern mit der Hinterlegungsnummer von In Vitro International, Inc. IVI-10102 (BBG-12), IVI-10103 (BBG-30), IVI-10104 (BBG-31) oder IVI-10105 (BBG-42) behandelt und
   b) bestimmt, ob eine Reaktion mit diesen Antikörpern eintritt.

6. Verfahren nach Anspruch 5, bei dem die Antikörper markiert sind und diese markierten Antikörper ein vorbestimmten Fluoreszenzansprechen auf eine bestimmte optische Stimulierung haben.

**7.** Verfahren nach Anspruch 1, bei dem die Reaktionsmenge bestimmt wird und das Vorhanden sein von Periodontopathogenen quantitativ daraus abgeleitet wird.

**8.** Verfahren nach Anspruch 5, bei dem die Bestimmung durch ELISA nach Lysis der Suspension durch eine löslichmachende Lösung erfolgt.

**Revendications**

**1.** Anticorps monoclonal capable de déceler un caractère de différenciation entre différents types de bactéries Bacteroïdes gingivalis et ayant le numéro de dépôt de In Vitro International Inc. IVI-10102 (BBG-12), IVI-10103 (BBG-30), IVI-10104 (BBG-31) ou IVI-10105 (BBG-42).

**2.** Anticorps monoclonal selon la revendication 1, dans lequel le caractère est le sérotype et l'anticorps monoclonal est IVI-10102 (BBG-12) ou IVI-10105 (BBG-42).

**3.** Anticorps monoclonal selon la revendication 1, dans lequel le caractère est la présence de fimbriae et l'anticorps monoclonal est IVI-10103 (BBG-30) ou IVI-10104 (BBG-31).

**4.** Procédé de détermination de la présence de types particuliers de Bacteroïdes gingivalis dans un fluide gingival, comprenant les étapes de
a) mise en contact du fluide gingival avec un anticorps monoclonal ayant le numéro de dépôt de In Vitro International Inc. IVI-10102 (BBG-12), IVI-10103 (BBG-30), IVI-10104 (BBG-31) ou IVI-10105 (BBG-42), pour la formation de complexes de ladite substance antigène et dudit anticorps monoclonal, et
b) mesure de la quantité dudit complexe par détermination de la quantité d'anticorps dans ledit complexe formé.

**5.** Procédé de détermination de la présence de types particuliers de Bacteroïdes gingivalis dans un fluide biologique, comprenant les étapes de
a) mise en contact d'une partie aliquote du fluide biologique à étudier avec une quantité déterminée d'anticorps ayant le numéro de dépôt de In Vitro international Inc. IVI-10102 (BBG-12), IVI-10103 (BBG-30), IVI-10104 (BBG-31) ou IVI-10105 (BBG-42), et
b) détermination de l'apparition de toute réaction avec lesdits anticorps.

**6.** Procédé selon la revendication 5, dans lequel les anticorps sont marqués et lesdits anticorps marqués ont une réponse de fluorescence préétablie à une stimulation optique donnée.

**7.** Procédé selon la revendication 5, dans lequel la quantité de réaction est déterminée et la présence de périodontopathogènes est déduite quantitativement de celle-ci.

**8.** Procédé selon la revendication 5, dans lequel ladite détermination est faite par essai immuno-enzymatique (ELISA) après lyse de la suspension par une solution de solubilisation.